# FASCICULE DE BREVET EUROPEEN

(11) **EP 1 848 987 B1**
(45) Date de publication et mention de la délivrance du brevet: **07.07.2010**
(21) Numéro de dépôt: 06708160.4
(22) Date de dépôt: 09.02.2006
(51) Int. Cl.: G01N 27/22, G01N 27/06, G01N 33/28

(54) **PROCEDE ET DISPOSITIF DE CONTROLE DE LA QUALITE DE L'HUILE D'UN MOTEUR THERMIQUE**
VERFAHREN UND VORRICHTUNG ZUR KONTROLLE DER QUALITÄT EINES WÄRMEKRAFTMASCHINENÖLS
METHOD AND DEVICE FOR CONTROLLING THE QUALITY OF A HEAT ENGINE OIL

(30) Priorité: 16.02.2005 FR 0501563
(43) Date de publication de la demande: 31.10.2007
(73) Titulaire: SC2N, 94000 Créteil (FR)
(72) Inventeur: WITTEMBERG, Vincent, F-14123 Ifs (FR)
(74) Mandataire: Croonenbroek, Thomas Jakob
(86) Numéro de dépôt international: PCT/EP2006/050816
(87) Numéro de publication internationale: WO 2006/087293

(56) Documents cités:
- EP-A- 1 426 755
- US-A1- 2003 222 656
- PATENT ABSTRACTS OF JAPAN vol. 014, no. 464 (M-1033), 9 octobre 1990 (1990-10-09) -& JP 02 185609 A (SUZUKI MOTOR CO LTD), 20 juillet 1990 (1990-07-20)

## Description

La présente invention concerne le domaine du contrôle de la qualité de l'huile d'un moteur thermique.

Plus précisément, la présente invention concerne la détermination de la dilution carburant dans l'huile moteur.

L'homme de l'art sait combien il est important de contrôler la qualité d'une huile moteur. De nombreux processus de contrôle à cette fin ont déjà été proposés. Cependant, aucun des processus connus ne donne totalement satisfaction.

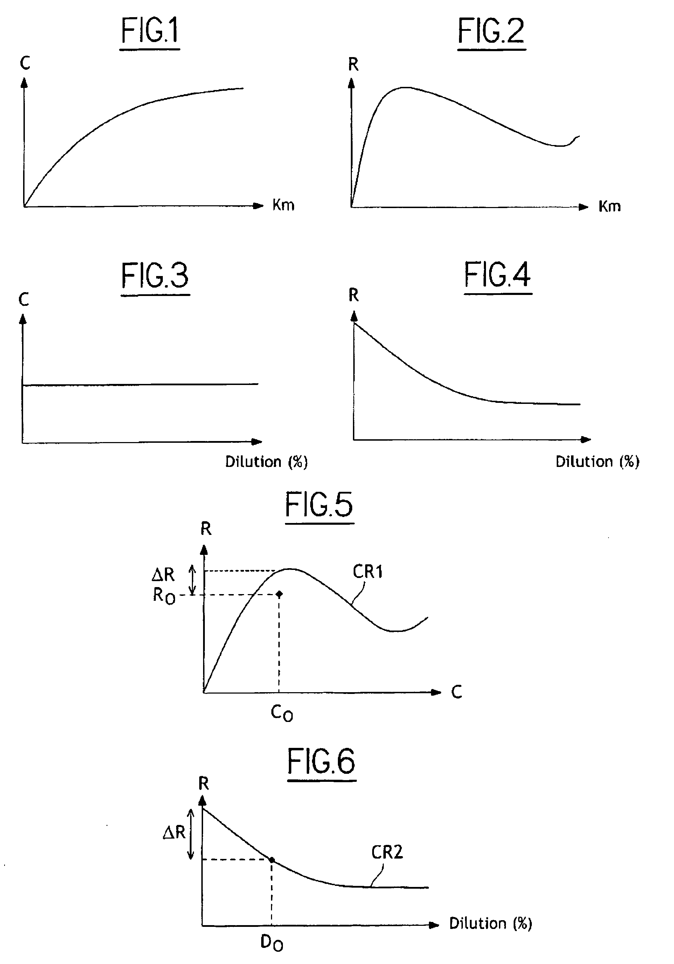

Il est bien connu en particulier que la valeur de la capacité électrique et de la résistance électrique d'une huile varie en fonction de son utilisation, notamment dans le cas d'un véhicule diesel. On a représenté sur les figures 1 et 2 annexées, des courbes typiques d'évolution de la valeur de la capacité électrique et de la résistance électrique de l'huile en fonction d'un kilométrage parcouru.

Voir aussi US 2003/223 656 A1 qui décrit de telles mesures.

Sur cette base, une méthode classique pour déterminer une limite de l'état de l'huile pour sa vidange est de fixer une limite sur la valeur de la capacité électrique.

Cette méthode est efficace dans le cas où l'huile est à vidanger à cause de son vieillissement classique. La mesure de la capacité qui sert de paramètre déterminant pour imposer la vidange prend en effet en compte entre autre, l'augmentation de la viscosité, l'état d'oxydation et la teneur en matière charbonneuse résultant de l'usure de l'huile.

En revanche, lorsqu'une dilution en carburant apparaît dans l'huile, la méthode précitée ne fonctionne pas.

En effet, la capacité électrique de l'huile est liée directement à sa permittivité diélectrique. Or, la permittivité diélectrique de l'huile et du carburant étant très proche, aucune variation sensible de la capacité électrique n'est obtenue en cas de dilution de carburant.

On a représenté à cette fin sur la figure 3 annexée, la capacité électrique d'une huile en fonction du pourcentage de dilution en carburant.

Par contre, les conductivités électriques de l'huile et du carburant étant très différentes, leur résistance électrique l'est également. Ainsi, comme illustré sur la figure 4, la résistance électrique de l'huile varie avec la dilution en carburant.

Cependant, jusqu'ici il n'a pas été possible d'exploiter correctement la courbe résultante, dans la mesure où la résistance varie non seulement en fonction de la dilution, mais également en fonction de l'usure de l'huile comme illustrée sur la figure 2.

La présente invention a pour but de proposer de nouveaux moyens permettant de déterminer la dilution en carburant dans une huile de moteur thermique.

Ce but est atteint dans le cadre de la présente invention, grâce à un procédé comprenant les étapes qui consistent à :
- mesurer la résistance électrique et la capacité électrique d'un échantillon d'huile à évaluer,
- comparer le couple de valeurs ainsi mesuré avec une première courbe de référence correspondant à l'évolution de la résistance électrique en fonction de la capacité électrique pour une huile sans dilution de carburant,
- relever l'écart entre la valeur de résistance électrique mesurée et la courbe de référence pour la valeur de capacité électrique mesurée,
- reporter l'écart de résistance électrique ainsi obtenu sur une seconde courbe de référence correspondant à l'évolution de la résistance électrique en fonction de la dilution en carburant, et
- retenir la valeur de dilution en carburant de la seconde courbe de référence qui correspond à l'écart de résistance ainsi reporté.

La présente invention concerne également un dispositif pour la mise en oeuvre du procédé précité.

D'autres caractéristiques, buts et avantages de la présente invention apparaîtront à la lecture de la description détaillée qui va suivre, et en regard des dessins annexés, donnés à titre d'exemples non limitatifs et sur lesquels :
- les figures 1, 2, 3 et 4 précédemment décrites représentent quatre courbes connues illustrant l'évolution de paramètres d'huile,
- la figure 5 représente une première courbe de référence, ou gabarit, exploitée selon la présente invention, et
- la figure 6 représente une seconde courbe de référence utilisée dans le cadre de la présente invention.

Comme on l'a évoqué précédemment, dans le cadre de la présente invention, le processus de détermination de la dilution en carburant d'une huile de moteur thermique comprend une première étape consistant à mesurer la résistance électrique R₀ et la capacité électrique C₀ d'un échantillon d'huile à analyser, à un instant donné.

Puis dans une seconde étape, le couple de valeurs R₀, C₀ ainsi mesuré est comparé avec une première courbe de référence CR1 illustrée sur la figure 5 qui correspond à l'évolution R = F (C) de la résistance R en fonction de la capacité électrique C d'une huile sans dilution en carburant correspondant à un vieillissement classique.

La troisième étape du procédé consiste à relever l'écart ΔR existant entre la valeur de résistance R₀ mesurée et la courbe de référence CR1 pour la valeur de capacité C₀ mesurée, comme illustrée sur la figure 5.

Dans une quatrième étape du procédé selon l'invention, l'écart de résistance ΔR précédemment obtenu est reporté sur une seconde courbe de référence CR2 illustrée sur la figure 6 correspondant à l'évolution de la résistance R en fonction de la dilution en carburant.

Enfin, dans le cadre de la présente invention, on retient comme valeur de dilution D₀, la valeur de la seconde courbe de référence CR2 qui correspond à l'écart de résistance ΔR, par rapport à l'origine, ainsi reporté.

En d'autres termes, dans le cadre de la présente invention, l'exploitation de la première courbe de référence CR1 illustré sur la figure 5 permet de ne retenir que l'écart de résistance ΔR par rapport au gabarit qui dépend de la dilution. Puis, la valeur de la dilution peut être obtenue par simple report du ΔR ainsi obtenu sur la courbe de la figure 6.

En pratique, les traitements d'informations précités conformes à la présente invention peuvent être opérés manuellement sous forme graphique ou à l'aide de tables ou abaques, voire automatiquement par tout moyen de calcul approprié exploitant des graphiques, des tables ou des traitements différents, tels que l'utilisation de logiques floues ou de réseaux de neurones.

Bien entendu la présente invention n'est pas limitée aux modes de réalisation particuliers qui viennent d'être décrits mais s'étend à toute variante conforme aux revendications suivantes.

## Revendications

1. Procédé permettant de déterminer la dilution en carburant d'une huile de moteur thermique, consistant à :
- mesurer la résistance électrique (R₀) et la capacité électrique (C₀) d'un échantillon d'huile à évaluer,
- comparer le couple de valeurs (R₀, C₀) ainsi mesuré avec une première courbe de référence (CR1) correspondant à l'évolution de la résistance (R) en fonction de la capacité (C) pour une huile sans dilution de carburant,
- relever l'écart (ΔR) entre la valeur de résistance mesurée (R₀) et la courbe de référence (CR2) pour la valeur de capacité mesurée (C₀),
- reporter l'écart de résistance (ΔR) ainsi obtenu sur une seconde courbe de référence (CR2) correspond à l'évolution de la résistance (R) en fonction de la dilution en carburant (D) et
- retenir la valeur de dilution (D₀) de la seconde courbe de référence (CR2) qui correspond à l'écart de résistance (ΔR) ainsi reporté.

2. Procédé selon la revendication 1, **caractérisé par le fait que** le traitement d'informations est opéré manuellement, par exemple sur graphiques, tables ou abaques.

3. Procédé selon la revendication 1, **caractérisé par le fait que** le traitement d'informations est opéré automatiquement, par exemple à l'aide de tables, logiques floues ou réseaux de neurones.

4. Dispositif permettant de déterminer la dilution en carburant d'une huile de moteur électrique, comprenant :
- des moyens permettant de mesurer la résistance électrique (R₀) et la capacité électrique (C₀) d'un échantillon d'huile à évaluer,
- des moyens permettant de comparer le couple de valeurs (R₀, C₀) ainsi mesuré avec une première courbe de référence (CR1) correspondant à l'évolution de la résistance (R) en fonction de la capacité électrique pour une huile sans dilution de carburant,
- des moyens permettant de relever l'écart (ΔR) entre la valeur de résistance mesurée (R₀) et la courbe de référence (CR1) pour la valeur de capacité mesurée (C₀),
- des moyens permettant de reporter l'écart de résistance (ΔR) ainsi obtenu sur une seconde courbe de référence (CR2) correspondant à l'évolution de la résistance (R) en fonction de la dilution (D), et
- des moyens permettant de retenir la valeur de dilution (D₀) de la seconde courbe de référence (CR2) qui correspond à l'écart de résistance (ΔR) ainsi reporté.

## Claims

1. Method for determining the dilution of an internal-combustion engine oil with fuel, consisting in:
- measuring the electrical resistance (R₀) and the electrical capacitance (C₀) of an oil sample to be evaluated;
- comparing the pairs of values (R₀, C₀) thus measured with a first reference curve (CR1) corresponding to the variation in the resistance (R) as a function of the capacitance (C) for an oil with no fuel dilution;
- recording the difference (ΔR) between the measured resistance value (R₀) and the reference curve (CR1) for the measured capacitance value (C₀);
- plotting the resistance difference (ΔR) thus obtained on a second reference curve (CR2) corresponding to the variation in the resistance (R) as a function of the fuel dilution (D); and
- taking the dilution value (D₀) of the second reference curve (CR2) that corresponds to the resistance difference (ΔR) thus plotted.

2. Method according to Claim 1, **characterized in that** the data processing is carried out manually, for example on graphs, tables or charts.

3. Method according to Claim 1, **characterized in that** the data processing is carried out automatically, for example using tables, fuzzy logic or neural networks.

4. Device for determining the dilution of an internal-combustion engine oil with fuel, comprising:
- means for measuring the electrical resistance (R₀) and the electrical capacitance (C₀) of an oil sample to be evaluated;
- means for comparing the pair of values (R₀, C₀) thus measured with a first reference curve (CR1) corresponding to the variation in the resistance (R) as a function of the electrical capacitance for an oil with no fuel dilution;
- means for taking the difference (ΔR) between the measured resistance value (R₀) and the reference curve (CR1) for the measured capacitance value (C₀);
- means for plotting the resistance difference (ΔR) thus obtained on a second reference curve (CR2) corresponding to the variation in the resistance (R) of the function of the dilution (D); and
- means for taking the dilution value (D₀) of the second reference curve (CR2) that corresponds to the resistance difference (ΔR) thus plotted.

## Patentansprüche

1. Verfahren, das es ermöglicht, die Verdünnung eines Verbrennungsmotorenöls durch Kraftstoff zu bestimmen, das darin besteht:
- den elektrischen Widerstand (R₀) und die elektrische Kapazität (C₀) einer zu bewertenden Ölprobe zu messen,
- das so gemessene Wertepaar (R₀, C₀) mit einer ersten Bezugskurve (CR1) zu vergleichen, die der Entwicklung des Widerstands (R) in Abhängigkeit von der Kapazität (C) für ein Öl ohne Kraftstoffverdünnung entspricht,
- die Abweichung (ΔR) zwischen dem gemessenen Widerstandswert (R₀) und der Bezugskurve (CR1) für den gemessenen Kapazitätswert (C₀) zu erfassen,
- die so erhaltene Widerstandsabweichung (ΔR) auf eine zweite Bezugskurve (CR2) zu übertragen, die der Entwicklung des Widerstands (R) in Abhängigkeit von der Verdünnung durch Kraftstoff (D) entspricht, und
- den Verdünnungswert (D₀) der zweiten Bezugskurve (CR2) zu speichern, der der so übertragenen Widerstandsabweichung (ΔR) entspricht.

2. Verfahren nach Anspruch 1, **dadurch gekennzeichnet, dass** die Datenverarbeitung manuell durchgeführt wird, zum Beispiel auf Grafiken, Tabellen oder Kurvenblättern.

3. Verfahren nach Anspruch 1, **dadurch gekennzeichnet, dass** die Datenverarbeitung automatisch durchgeführt wird, zum Beispiel mit Hilfe von Tabellen, Fuzzy-Logiken oder neuronalen Netzen.

4. Vorrichtung, die es ermöglicht, die Verdünnung eines Verbrennungsmotorenöls durch Kraftstoff zu bestimmen, die enthält:
- Einrichtungen, die es ermöglichen, den elektrischen Widerstand (R₀) und die elektrische Kapazität (C₀) einer zu bewertenden Ölprobe zu messen,
- Einrichtungen, die es ermöglichen, das so gemessene Wertepaar (R₀, C₀) mit einer ersten Bezugskurve (CR1) zu vergleichen, die der Entwicklung des Widerstands (R) in Abhängigkeit von der elektrischen Kapazität für ein Öl ohne Kraftstoffverdünnung entspricht,
- Einrichtungen, die es ermöglichen, die Abweichung (ΔR) zwischen dem gemessenen Widerstandswert (R₀) und der Bezugskurve (CR1) für den gemessenen Kapazitätswert (C₀) zu erfassen,
- Einrichtungen, die es ermöglichen, die so erhaltene Widerstandsabweichung (ΔR) auf eine zweite Bezugskurve (CR2) zu übertragen, die der Entwicklung des Widerstands (R) in Abhängigkeit von der Verdünnung (D) entspricht, und
- Einrichtungen, die es ermöglichen, den Verdünnungswert (D₀) der zweiten Bezugskurve (CR2) zu speichern, der der so übertragenen Widerstandsabweichung (ΔR) entspricht.
